(19) Europäisches Patentamt
European Patent Office
Office européen des brevets

(11) **EP 1 985 600 B1**

(12) **FASCICULE DE BREVET EUROPEEN**

(45) Date de publication et mention
de la délivrance du brevet:
**06.05.2020 Bulletin 2020/19**

(51) Int Cl.:
***C07C 5/27*** (2006.01)  ***B01J 29/74*** (2006.01)

(21) Numéro de dépôt: **08290312.1**

(22) Date de dépôt: **31.03.2008**

(54) **Procédé d'isomérisation d'une coupe C8 aromatique en présence d'un catalyseur à base d'une zéolithe EUO désaluminée**

Isomerisationsverfahren einer aromatischen C8-Fraktion in Gegenwart eines Katalysators auf der Basis eines entaluminierten EUO-Zeoliths

Method for isomerising an aromatic C8 fraction in the presence of a catalyst made from a dealuminated EUO zeolite

(84) Etats contractants désignés:
**BE DE GB NL**

(30) Priorité: **23.04.2007 FR 0702941**

(43) Date de publication de la demande:
**29.10.2008 Bulletin 2008/44**

(73) Titulaire: **IFP Energies nouvelles
92500 Rueil-Malmaison (FR)**

(72) Inventeurs:
• **Guillon, Emmanuelle
69390 Vernaison (FR)**
• **Sanchez, Eric
69230 Saint Genis Laval (FR)**

(74) Mandataire: **IFP Energies nouvelles
Département Propriété Industrielle
Rond Point de l'échangeur de Solaize
BP3
69360 Solaize (FR)**

(56) Documents cités:
**EP-A- 0 923 987     EP-A1- 0 363 253
EP-A1- 0 999 182     FR-A- 2 765 209
US-A- 4 695 667      US-B1- 6 313 363**

• **RAO G N ET AL: "THERMAL AND HYDROTHERMAL STABILITIES OF ZEOLITE EU-1" APPLIED CATALYSIS A: GENERAL, ELSEVIER SCIENCE, AMSTERDAM, NL, vol. 119, 1994, pages 33-43, XP002055790 ISSN: 0926-860X**

**Description**

Domaine de l'invention

La présente invention se rapporte à l'isomérisation d'une coupe aromatique contenant au moins un composé aromatique à huit atomes de carbone par molécule à une température comprise entre 300 et 500°C, une pression partielle d'hydrogène comprise entre 0,3 et 1,5 MPa, une pression totale comprise entre 0,45 et 1,9 MPa et une vitesse spatiale d'alimentation, exprimée en kilogramme de charge introduite par kilogramme de catalyseur et par heure, comprise entre 0,25 et 30 h-1, en vue de la production de xylènes. Ladite coupe aromatique visée dans la présente invention est une charge contenant un mélange de xylènes, de l'éthylbenzène ou un mélange de xylènes et d'éthylbenzène. Cette charge est communément appelée "coupe C8 aromatique".
La présente invention concerne plus particulièrement un procédé d'isomérisation d'une charge aromatique comprenant au moins un composé aromatique à huit atomes de carbone par molécule visant à maximiser la production de para-xylène.

Etat de la technique antérieure

La catalyse de l'isomérisation de l'éthylbenzène en xylènes nécessite la présence d'un métal du groupe VIII. Les formulations optimisées à base de mordénite et d'un métal du groupe VIII conduisent à des catalyseurs avec lesquels les réactions parasites restent non négligeables. Par exemple, on peut citer l'ouverture de cycles naphténiques, suivie ou non de craquage, ou encore les réactions de dismutation et de transalkylation des aromatiques en C8, qui conduisent à la formation d'aromatiques non recherchés. Il est donc particulièrement intéressant de trouver de nouveaux catalyseurs plus sélectifs.
Parmi les zéolithes utilisées en isomérisation des coupes C8 aromatiques, on trouve la ZSM-5, utilisée seule ou en mélange avec d'autres zéolithes, comme par exemple la mordénite. Ces catalyseurs sont notamment décrits dans les brevets US-A-4 467 129, US-A-4 482 773 et EP-B-13 617. D'autres catalyseurs principalement à base de mordénite ont été décrits par exemple dans les brevets US-A-4 723 051, US-A-4 665 258 et FR-A-2 477 903. Plus récemment, il a été proposé un catalyseur à base d'une zéolithe de type structural EUO (EP-A1-923 987). La demande de brevet WO-A-2005/065 380 décrit l'utilisation d'une zéolithe de type structural MTW en isomérisation des xylènes et de l'éthylbenzène.

Résumé et intérêt de l'invention

La présente invention concerne un procédé d'isomérisation d'une coupe aromatique contenant au moins un composé aromatique ayant huit atomes de carbone par molécule à une température comprise entre 300 et 500°C, une pression partielle d'hydrogène comprise entre 0,3 et 1,5 MPa, une pression totale comprise entre 0,45 et 1,9 MPa et une vitesse spatiale d'alimentation, exprimée en kilogramme de charge introduite par kilogramme de catalyseur et par heure, comprise entre 0,25 et 30 h-1, comprenant la mise en contact de ladite coupe avec au moins un catalyseur contenant au moins une zéolithe de type structural EUO, ledit catalyseur ayant été préparé selon un procédé comprenant au moins les étapes suivantes :

i) la synthèse d'au moins une zéolithe de type structural EUO ayant un rapport atomique Si/Al global compris entre 5 et 45,
ii) la désalumination de la zéolithe obtenue à l'issue de ladite étape i), par au moins un traitement par une solution aqueuse d'un acide minéral ou d'un acide organique, de telle manière qu'au moins 10% massique des atomes d'aluminium soient extraits de ladite zéolithe issue de ladite étape i),
iii) la mise en forme de ladite zéolithe désaluminée avec une matrice,
iv) le dépôt d'au moins un métal du groupe VIII de la classification périodique des éléments, l'ordre de réalisation desdites étapes iii) et iv) étant indifférent à la suite de ladite étape ii).

Il a été découvert, de façon surprenante, qu'un catalyseur, sous forme d'extrudés ou de billes, comprenant au moins une zéolithe de type structural EUO désaluminée de sorte qu'au moins 10% massique des atomes d'aluminium de la zéolithe de type structural EUO sous sa forme brute de synthèse aient été extraits, au moins une matrice et au moins un métal du groupe VIII de la classification périodique des éléments conduit à des performances catalytiques améliorées en terme de sélectivité lorsqu'il est utilisé dans un procédé d'isomérisation d'une coupe aromatique comprenant au moins un composé aromatique à huit atomes de carbone par molécule. En particulier, un tel catalyseur est plus sélectif envers les produits recherchés, à savoir les xylènes et en particulier le para-xylène, qu'un catalyseur de l'état de la technique antérieure à base d'une zéolithe de type structural EUO non désaluminée. Cette sélectivité accrue envers l'isomérisation des xylènes est au détriment des réactions parasites, non désirées, de craquage, désalkylation, transalkylation et de dismutation. La désalumination de la zéolithe obtenue à l'issue de ladite étape i) du procédé de l'invention est réalisée par au moins un traitement par une solution aqueuse d'un acide minéral ou d'un acide organique.

Description de l'invention

La présente invention a pour objet un procédé d'isomérisation d'une coupe aromatique contenant au moins un composé aromatique ayant huit atomes de carbone par molécule à une température comprise entre 300 et 500°C, une pression partielle d'hydrogène comprise entre 0,3 et 1,5 MPa, une pression totale comprise entre 0,45 et 1,9 MPa et une vitesse spatiale d'alimentation, exprimée en kilogramme de charge introduite par kilogramme de catalyseur et par heure, comprise entre 0,25 et 30 h-1, comprenant la mise en contact de ladite coupe avec au moins un catalyseur contenant au moins une zéolithe de type structural EUO, ledit catalyseur ayant été préparé selon un procédé comprenant au moins les étapes suivantes :

i) la synthèse d'au moins une zéolithe de type structural EUO ayant un rapport atomique Si/Al global compris entre 5 et 45,
ii) la désalumination de la zéolithe obtenue à l'issue de ladite étape i), par au moins un traitement par une solution aqueuse d'un acide minéral ou d'un acide organique, de telle manière qu'au moins 10% massique des atomes d'aluminium soient extraits de ladite zéolithe issue de ladite étape i),
iii) la mise en forme de ladite zéolithe désaluminée avec une matrice,
iv) le dépôt d'au moins un métal du groupe VIII de la classification périodique des éléments, l'ordre de réalisation desdites étapes iii) et iv) étant indifférent à la suite de ladite étape ii).

La zéolithe de type structural EUO, désaluminée par au moins un traitement par une solution aqueuse d'un acide minéral ou d'un acide organique, présente dans le catalyseur utilisé pour la mise en oeuvre du procédé d'isomérisation selon l'invention est une zéolithe choisie parmi la zéolithe EU-1, la zéolithe TPZ-3 et la zéolithe ZSM-50, de préférence il s'agit d'une zéolithe EU-1. Les zéolithes EU-1, TPZ-3 et ZSM-50 de type structural EUO sont bien connues de l'art antérieur (Atlas of Zeolite Framework Types, Ch. Baerlocher, W.M. Meier, D.H. Oison, 5ème édition, 2001). Il est connu qu'une zéolithe de type structural EUO, en particulier une zéolithe EU-1, présente un réseau microporeux monodimensionnel, dont le diamètre des pores est de 4,1 x 5,4 Å (1 Å = 1 Angström = $10^{-10}$ m). D'autre part, N.A. Briscoe et al ont enseigné dans un article de la revue Zeolites (1988, 8, 74) que ces canaux monodimensionnels possèdent des poches latérales de profondeur 8,1 Å et de diamètre 6,8 x 5,8 Å.
Le mode de préparation des différentes zéolithes de type structural EUO est également bien connu de l'Homme du métier. De manière générale, les méthodes de préparation de telles zéolithes comprennent le mélange en milieu aqueux d'une source de silicium, d'une source d'aluminium, d'une source d'un métal alcalin et d'un composé organique azoté jouant le rôle de structurant. La zéolithe EU-1, décrite dans la demande de brevet européenne EP-A-0 042 226, est préparée en utilisant comme structurant soit le dérivé alkylé d'une polyméthylène $\alpha$-$\omega$ diammonium, soit un produit de dégradation dudit dérivé soit encore des précurseurs dudit dérivé. La zéolithe TPZ-3, décrite dans la demande de brevet européenne EP-A-0 051 318, est préparée en utilisant la même famille de structurant que celle employée pour synthétiser la zéolithe EU-1. Il est notamment décrit l'utilisation du composé 1,6-N,N,N,N',N',N'-hexaméthylhexaméthylènediammonium. La zéolithe ZSM-50, décrite dans les documents EP 0 159 845 et US-A-4,640,829, est préparée en utilisant comme structurant le dérivé dibenzyldiméthylammonium (DBDMA). Aussi, pour la mise en oeuvre de ladite étape i) de préparation de la zéolithe de type structural EUO présente dans le catalyseur employé dans le procédé d'isomérisation selon l'invention, l'Homme du métier se référera utilement à l'une ou l'autre des références citées ci-dessus décrivant la préparation de telles zéolithes.
Plus précisément, pour la préparation d'une zéolithe EU-1 selon ladite étape i), on mélange en milieu aqueux au moins une source de silicium, au moins une source d'aluminium, au moins un structurant organique azoté Q de formule $R_1R_2R_3$ - $N^+$ - $(CH_2)_n$ - $N^+$ - $R_4R_5R_6$, dans laquelle n est compris entre 3 et 12, les groupes $R_1$ à $R_6$, identiques ou différents, sont des groupes alkyles ayant de 1 à 8 atomes de carbone, jusqu'à cinq desdits groupes $R_1$ à $R_6$ pouvant être de l'hydrogène, éventuellement des germes zéolithiques.
Le mélange réactionnel a la composition molaire suivante :

| | |
|---|---|
| $SiO_2/Al_2O_3$ | 10 - 150 |
| $OH^-/SiO_2$ | 0,1 - 6 |
| $(M^+ + Q)/Al_2O_3$ | 0,5 - 100 |
| $Q/(M^+ + Q)$ | 0,1 - 10 |
| $H_2O/SiO_2$ | 1 - 100, |

Q étant le cation $R_1R_2R_3 - N^+ - (CH_2)_n - N^+ - R_4R_5R_6$, décrit ci-dessus, de préférence le 1,6 N,N,N,N',N',N'-hexaméthylhexaméthylène diammonium et $M^+$ étant un cation alcalin ou l'ammonium.

Ledit mélange réactionnel est mis à réagir sous la pression autogène, éventuellement avec un apport d'un gaz, par exemple de l'azote, à une température comprise entre 85 et 250°C jusqu'à ce que les cristaux de zéolithes EU-1 se forment. La durée de la réaction est comprise entre 1 minute et plusieurs mois suivant la composition des réactifs, le mode de chauffage et de mélange, la température de la réaction et l'agitation. Au terme de la réaction, la phase solide est collectée sur un filtre et lavée. A ce stade là, la zéolithe EU-1 est dite brute de synthèse et contient dans sa porosité intracristalline au moins le cation $R_1R_2R_3 - N^+ - (CH_2)_n - N^+ - R_4R_5R_6$, de préférence le 1,6 N,N,N,N',N',N'-hexaméthylhexaméthylène diammonium. Conformément à l'invention, ladite zéolithe EU-1 brute de synthèse, obtenue à l'issue de l'étape i), présente un rapport atomique Si/Al global compris entre 5 et 45, de préférence entre 10 et 40 et de manière encore plus préférée entre 10 et 25. Le rapport atomique Si/Al global, déterminé par fluorescence X ou absorption atomique, prend en compte aussi bien les atomes d'aluminium présents dans la charpente zéolithique que les atomes d'aluminium éventuellement présents hors de ladite charpente zéolithique encore appelés aluminium extra-réseau.

L'étape ii) du procédé de préparation de la zéolithe de type structural EUO, de préférence de la zéolithe EU-1, présente dans le catalyseur employé pour la mise en oeuvre du procédé d'isomérisation selon l'invention, consiste à extraire au moins 10% massique, de préférence au moins 20% massique, des atomes d'aluminium de ladite zéolithe de type structural EUO, de préférence de la zéolithe EU-1, issue de ladite étape i) et se trouvant sous sa forme brute de synthèse. En conséquence, la zéolithe de type structural EUO désaluminée, de préférence la zéolithe EU-1 désaluminée, obtenue à l'issue de ladite étape ii) présente un rapport atomique Si/Al global supérieur à celui de la zéolithe de type structural EUO brute de synthèse, de préférence de la zéolithe EU-1 brute de synthèse, laquelle n'est pas encore désaluminée. L'étape ii) du procédé de préparation du catalyseur est réalisée en soumettant la zéolithe obtenue à l'issue de ladite étape i) à au moins un traitement par une solution aqueuse d'un acide minéral ou d'un acide organique.

Selon un premier mode de réalisation de ladite étape ii) de désalumination, la zéolithe de type structural EUO, de préférence la zéolithe EU-1, issue de ladite étape i) est soumise à une calcination sous flux d'air sec, à une température comprise entre 400 et 600°C puis est soumise à au moins un traitement par une solution aqueuse d'un acide minéral ou d'un acide organique. La durée de la calcination est variable et est comprise entre plusieurs heures et plusieurs jours. Le traitement par calcination de ladite zéolithe de type structural EUO issue de ladite étape i) a pour but d'éliminer le structurant organique présent dans la microporosité de la zéolithe, par exemple le cation $R_1R_2R_3 - N^+ - (CH_2)_n - N^+ - R_4R_5R_6$, de préférence le 1,6 N,N,N,N',N',N'-hexaméthylhexaméthylène diammonium lorsque la zéolithe synthétisée au cours de ladite étape i) est la zéolithe EU-1. Le pourcentage massique de carbone résiduel de la zéolithe à l'issue de ladite étape de calcination est de préférence inférieur à 0,3 % et de manière plus préférée inférieure à 0,1 %.

Ledit traitement de la zéolithe de type structural EUO, de préférence de la zéolithe EU-1, par une solution aqueuse d'un acide minéral ou organique effectué à la suite de l'étape de calcination est aussi appelé "étape d'attaque acide". Ledit traitement peut être répété autant de fois qu'il est nécessaire afin d'obtenir le niveau de désalumination voulu. Dans ce cas, la zéolithe est lavée à l'eau distillée entre chaque étape d'attaque acide successive.

De manière préférée, un ou plusieurs échange(s) ionique(s) par au moins une solution $NH_4NO_3$ est(sont) opéré(s) entre la calcination sous flux d'air sec et le traitement par la solution aqueuse acide de manière à éliminer au moins en partie, de préférence pratiquement totalement, le cation alcalin, en particulier le sodium, éventuellement présent en position cationique dans la zéolithe sous sa forme brute de synthèse. Chaque échange est réalisé à une température préférentiellement comprise entre 50 et 150°C pendant une durée avantageusement comprise entre 2 heures et 10 heures. On utilise généralement une solution aqueuse de nitrate d'ammonium $NH_4NO_3$ ayant une normalité comprise entre 7 et 12N. De même, à la fin de la mise en oeuvre de ladite étape ii) de désalumination lorsque le(s) traitement(s) par une solution aqueuse acide est(sont) réalisé(s), il est possible de réaliser un ou plusieurs échange(s) ionique(s) par au moins une solution $NH_4NO_3$, de manière à éliminer les cations alcalins résiduels et en particulier le sodium.

Pour que la zéolithe issue de ladite étape ii) présente le rapport atomique Si/Al global désiré après extraction d'au moins 10% massique des atomes d'aluminium de la zéolithe de type structural EUO issue de ladite étape i), il est nécessaire de bien choisir et contrôler les conditions opératoires de chaque étape d'attaque acide. En particulier, la température à laquelle le traitement par la solution aqueuse de l'acide minéral ou organique est réalisé, la nature et la concentration de l'acide utilisé, le rapport entre la quantité de solution acide et le poids de zéolithe traitée, la durée du traitement et le nombre de traitement réalisés sont des paramètres significatifs pour la mise en oeuvre de chaque étape d'attaque acide. De manière avantageuse, le traitement de la zéolithe par une solution aqueuse d'un acide minéral ou d'un acide organique est réalisé à une température comprise entre 30°C et 120°C, de préférence entre 50°C et 120°C, de manière très préférée entre 60 et 100°C. La concentration de l'acide dans la solution aqueuse est généralement comprise entre 0,05 et 20 mol.L$^{-1}$, de préférence entre 0,1 et 10 mol.L$^{-1}$, de manière encore plus préférée entre 0,5 et 5 mol.L$^{-1}$. Le rapport entre le volume de solution acide V en ml et le poids de zéolithe traitée P en g est généralement compris entre 1 et 50, de préférence entre 2 et 20. La durée de l'attaque acide est généralement supérieure à 1 heure, souvent comprise entre 2 heures et 10 heures, de préférence entre 2 heures et 8 heures. L'acide choisi pour la mise en oeuvre de ladite étape d'attaque acide est soit un acide minéral soit un acide organique, de préférence il s'agit d'un acide minéral choisi parmi l'acide nitrique HNO$_3$, l'acide chlorhydrique HCl et l'acide sulfurique H$_2$SO$_4$. De manière très préférée, il s'agit de l'acide nitrique. Lorsqu'un acide organique est utilisé pour l'attaque acide, l'acide acétique CH$_3$CO$_2$H est préféré. Le nombre de traitement successif de la zéolithe par une solution aqueuse acide est préférentiellement inférieur à 4. Dans le cas où plusieurs attaques acides successives sont réalisées, des solutions aqueuses d'acide minéral ou organique de concentrations différentes en acide peuvent être utilisées.

Après avoir effectué le(s) traitement(s) par la solution aqueuse acide, la zéolithe est ensuite lavée à l'eau distillée puis est séchée à une température comprise entre 80 et 140°C pendant une durée comprise entre 10 et 48 heures.

Cette méthode de désalumination réalisée conformément audit premier mode de réalisation de l'étape ii) du procédé de préparation de la zéolithe de type structural EUO présente dans le catalyseur employé pour la mise en oeuvre du procédé d'isomérisation selon l'invention est dite méthode de l'attaque acide directe.

Selon un deuxième mode de réalisation de ladite étape ii) de désalumination, la zéolithe de type structural EUO, de préférence la zéolithe EU-1, issue de ladite étape i) est soumise à une calcination sous flux d'air sec, à une température comprise entre 400 et 600°C puis à un ou plusieurs échange(s) ionique(s) par au moins une solution NH$_4$NO$_3$ puis est soumise à au moins un cycle de désalumination de la charpente zéolithique comportant au moins un traitement thermique réalisé en présence de vapeur d'eau et au moins une attaque acide par au moins une solution aqueuse d'un acide minéral ou organique.

La durée de la calcination sous flux d'air sec est variable et est comprise entre plusieurs heures et plusieurs jours. Le traitement par calcination de la zéolithe de type structural EUO issue de ladite étape i) a pour but d'éliminer le structurant organique présent dans la microporosité de ladite zéolithe, par exemple le cation R$_1$R$_2$R$_3$ - N$^+$ - (CH$_2$)$_n$ - N$^+$ - R$_4$R$_5$R$_6$, de préférence le 1,6 N,N,N,N',N',N'-hexaméthylhexaméthylène diammonium lorsque la zéolithe synthétisée au cours de ladite étape i) est la zéolithe EU-1. Le ou les échange(s) ionique(s) subséquent(s) à ladite calcination sous flux d'air sec permet(tent) d'éliminer au moins en partie, de préférence pratiquement totalement, le cation alcalin, en particulier le sodium, éventuellement présent en position cationique dans la zéolithe sous sa forme brute de synthèse. Chaque échange est réalisé à une température préférentiellement comprise entre 50 et 150°C pendant une durée avantageusement comprise entre 2 heures et 10 heures. On utilise généralement une solution aqueuse de nitrate d'ammonium NH$_4$NO$_3$ ayant une normalité comprise entre 7 et 12N.

Les conditions opératoires du traitement thermique en présence de vapeur d'eau, en particulier la température et la durée dudit traitement ainsi que le pourcentage volumique de vapeur d'eau, de même que les conditions opératoires de l'attaque acide post-traitement thermique, en particulier la durée de l'attaque acide, la nature et la concentration de l'acide utilisé, et le rapport entre le volume de solution acide et le poids de zéolithe traitée, sont adaptées de manière à obtenir une zéolithe de type structural EUO désaluminée, de préférence une zéolithe EU-1 désaluminée, présentant le rapport atomique Si/Al global désiré après l'extraction d'au moins 10% massique des atomes d'aluminium présents dans la zéolithe brute de synthèse issue de ladite étape i). De manière avantageuse, le traitement thermique en présence de vapeur d'eau est réalisé à une température comprise entre 200 et 900°C, de préférence entre 300 et 900°C, de manière encore plus préférée entre 400 et 600°C. La durée dudit traitement thermique est généralement supérieure ou égale à 0,5 heure, préférentiellement comprise entre 0,5 heure et 24 heures, et très préférentiellement comprise entre 0,5 heure et 12 heures. Le pourcentage volumique de vapeur d'eau durant le traitement thermique est généralement compris entre 5 et 100%, de préférence entre 20 et 100%, de manière encore plus préférée entre 40% et 100%. La fraction volumique autre que la vapeur d'eau éventuellement présente est formée d'air. Le débit de gaz formé de vapeur d'eau et éventuellement d'air est compris entre 0,2 l/h/g de solide traité et 10 l/h/g de solide traité.

La température à laquelle est réalisée l'attaque acide, subséquente au traitement thermique en présence de vapeur d'eau, est généralement comprise entre 30°C et 120°C, de préférence entre 50 et 120°C, de manière très préférée entre 60 et 100°C. La concentration de l'acide dans la solution aqueuse est généralement comprise entre 0,05 et 20 mol.L$^{-1}$, de préférence entre 0,1 et 10 mol.L$^{-1}$, de manière encore plus préférée entre 0,5 et 5 mol.L$^{-1}$. Le rapport entre le volume de solution aqueuse acide V en ml et le poids de zéolithe traitée P en g est généralement compris entre 1 et 50, de préférence entre 2 et 20. La durée de l'attaque acide est généralement supérieure à 1 heure, souvent comprise entre 2 heures et 10 heures, de préférence entre 2 heures et 8 heures. L'acide choisi pour la mise en oeuvre de l'attaque acide est soit un acide minéral soit un acide organique, de préférence il s'agit d'un acide minéral choisi parmi l'acide nitrique HNO$_3$, l'acide chlorhydrique HCl et l'acide sulfurique H$_2$SO$_4$. De manière très préférée, il s'agit de l'acide nitrique. Lorsqu'un acide organique est utilisé pour l'attaque acide, l'acide acétique CH$_3$CO$_2$H est préféré.

Le cycle de désalumination de la charpente zéolithique comportant au moins un traitement thermique réalisé en présence de vapeur d'eau et au moins une attaque acide par au moins une solution aqueuse d'un acide minéral ou organique peut être répété autant de fois qu'il est nécessaire pour obtenir la zéolithe de type structural EUO désaluminée, de préférence la zéolithe EU-1 désaluminée, possédant les caractéristiques désirées en particulier un rapport atomique Si/Al global supérieur à celui de la zéolithe sous sa forme brute de synthèse après extraction d'au moins 10% massique, de préférence d'au moins 20% massique, des atomes d'aluminium de la zéolithe brute de synthèse. Le nombre de cycle de désalumination est préférentiellement inférieur à 4.

Cette méthode de désalumination réalisée conformément audit deuxième mode de réalisation de l'étape ii) du procédé de préparation de la zéolithe de type structural EUO présente dans le catalyseur employé pour la mise en oeuvre du procédé d'isomérisation selon l'invention est dite méthode de traitement thermique et d'attaque acide.

Selon un troisième mode de réalisation de ladite étape ii) de désalumination, la zéolithe de type structural EUO, de préférence la zéolithe EU-1, issue de ladite étape i) est soumise à un traitement thermique réalisé en présence de vapeur d'eau à une température comprise entre 450 et 850°C, puis à au moins un traitement par une solution aqueuse d'un acide minéral ou organique. Le(s)dit(s) traitement(s) par une solution aqueuse acide est(sont) préférentiellement suivi(s) d'un ou plusieurs échange(s) ionique(s) par au moins une solution $NH_4NO_3$ de manière à éliminer pratiquement tout cation alcalin, en particulier le sodium, éventuellement présent en position cationique dans la zéolithe sous sa forme brute de synthèse. Chaque échange est réalisé à une température préférentiellement comprise entre 50 et 150°C pendant une durée avantageusement comprise entre 2 heures et 10 heures. On utilise généralement une solution aqueuse de nitrate d'ammonium $NH_4NO_3$ ayant une normalité comprise entre 7 et 12 N.

Conformément audit troisième mode de réalisation de ladite étape ii), ledit traitement thermique en présence de vapeur d'eau est réalisé simultanément à l'élimination du structurant organique, préférentiellement du cation $R_1R_2R_3$ - $N^+$ - $(CH_2)_n$ - $N^+$ - $R_4R_5R_6$, et très préférentiellement du 1,6 N,N,N,N',N',N'-hexaméthylhexaméthylène diammonium, présent dans la microporosité de ladite zéolithe. En effet, le traitement thermique en présence de vapeur d'eau est effectué à une température suffisamment élevée pour permettre l'élimination dudit structurant. Les conditions opératoires pour la mise en oeuvre dudit traitement thermique en présence de vapeur d'eau sont identiques à celles données ci-dessus pour la réalisation du traitement thermique en présence de vapeur d'eau mis en oeuvre dans le cycle de désalumination du deuxième mode de réalisation de l'étape ii) de désalumination. Le traitement par une solution aqueuse d'un acide minéral ou organique est réalisé dans les mêmes conditions opératoires que celles données ci-dessus pour la réalisation de l'attaque acide par au moins une solution aqueuse d'un acide minéral ou organique mise en oeuvre dans le cycle de désalumination du deuxième mode de réalisation de l'étape ii) de désalumination. Suite au traitement thermique réalisé en présence de vapeur d'eau, plusieurs attaques acides successives peuvent être opérées de manière à obtenir le niveau de désalumination voulu. Les solutions aqueuses acides utilisées pour la mise en oeuvre de ces différentes étapes d'attaque acide ont une concentration identique ou différente, préférentiellement différente. Entre chaque attaque acide, la zéolithe est lavée à l'eau distillée.

A l'issue de ladite étape ii) de désalumination, mise en oeuvre en soumettant la zéolithe obtenue à l'issue de ladite étape i) à au moins un traitement par une solution aqueuse d'un acide minéral ou d'un acide organique selon le premier, le deuxième ou le troisième mode de réalisation décrit ci-dessus, au moins 10% massique des atomes d'aluminium, de préférence au moins 20% massique des atomes d'aluminium, présents dans la zéolithe de type structural EUO brute de synthèse, de préférence dans la zéolithe EU-1 brute de synthèse, ont été extraits. La zéolithe de type structural EUO désaluminée, de préférence la zéolithe EU-1 désaluminée, obtenue à l'issue de ladite étape ii) présente une cristallinité supérieure à 85%, de préférence supérieure à 90%, de manière très préférée supérieure à 97%. La cristallinité est calculée à partir du diagramme de diffraction par comparaison avec une zéolithe de type structural EUO de référence. La cristallinité correspond au rapport de la surface des pics des solides analysés sur la surface des pics de la zéolithe de type structural EUO de référence dans le domaine d'angle de diffraction $2\theta$ = 8 à 40°. La zéolithe de type structural EUO désaluminée, de préférence la zéolithe EU-1 désaluminée, obtenue à l'issue de ladite étape ii) est dépourvue de mésopores : aucune cavité mésoporeuse ayant une taille comprise entre 2 et 50 nm n'est créée à la suite de la mise en oeuvre de l'étape ii) de désalumination.

Après la mise en oeuvre de l'étape ii) de désalumination, la zéolithe de type structural EUO désaluminée, de préférence la zéolithe EU-1 désaluminée, est ensuite lavée à l'eau puis est séchée à une température comprise entre 80 et 140°C pendant une durée comprise entre 10 et 48 heures.

La préparation du catalyseur comprenant une zéolithe de type structural EUO désaluminée, de préférence une zéolithe EU-1 désaluminée, en vue de son utilisation dans le procédé d'isomérisation selon l'invention, se poursuit par la mise en oeuvre de ladite étape iii) de mise en forme et par la mise en oeuvre de ladite étape iv) de dépôt d'au moins un métal du groupe VIII de la classification périodique des éléments. L'ordre de réalisation desdites étapes iii) et iv), subséquentes à ladite étape ii) est indifférent. De préférence, ladite étape iii) précède ladite étape iv).

Pour la mise en oeuvre de ladite étape iii) de mise en forme de ladite zéolithe de type structural EUO désaluminée, de préférence de la zéolithe EU-1 désaluminée, on utilise une matrice choisie parmi les argiles, la magnésie, les alumines, les silices, l'oxyde de titane, l'oxyde de bore, la zircone, les phosphates d'aluminium, les phosphates de titane, les phosphates de zirconium, les silices-alumines et le charbon ou un mélange d'au moins deux de ces compositions. De préférence, la matrice est une alumine. Avantageusement, la zéolithe associée à la matrice est mise sous forme de billes ou d'extrudés, très avantageusement sous forme d'extrudés.

La mise en forme conformément à ladite étape iii) consiste plus particulièrement à malaxer la zéolithe de type structural EUO désaluminée, de préférence la zéolithe EU-1 désaluminée, dans un gel humide de matrice, préférentiellement d'alumine, obtenu généralement par mélange d'au moins un acide et d'une poudre de matrice, pendant une durée nécessaire à l'obtention d'une bonne homogénéité de la pâte, soit par exemple pendant une dizaine de minutes, puis à passer la pâte ainsi obtenue à travers une filière pour former des extrudés, par exemple de diamètre de 0,4 à 4 mm.

La mise en forme est généralement suivie d'un séchage puis d'une calcination. Le séchage est avantageusement réalisé à une température comprise entre 100 et 150°C pendant une durée comprise entre 5 et 20 heures en étuve. La calcination est avantageusement réalisé à une température comprise entre 250°C et 600°C pendant une durée comprise entre 1 et 8 heures.

L'étape iv) de préparation du catalyseur comprenant une zéolithe de type structural EUO désaluminée, de préférence une zéolithe EU-1 désaluminée, consiste à introduire au moins un métal du groupe VIII de la classification périodique des éléments et éventuellement au moins un métal choisi parmi les métaux des groupes IIIA, IVA et VIIB. Ledit métal du groupe VIII présent dans le catalyseur utilisé dans le procédé d'isomérisation selon l'invention est choisi parmi le fer, le cobalt, le nickel, le ruthénium, le rhodium, le palladium, l'osmium, l'iridium et le platine, de préférence parmi les métaux nobles et très préférentiellement parmi le palladium et le platine. De manière encore plus préférée, ledit métal du groupe VIII est le platine. Selon la méthode mise en œuvre pour le dépôt dudit métal du groupe VIII, comme il est indiqué ci-après dans la description, ledit métal du groupe VIII, de préférence le platine, peut être déposé de manière prépondérante sur la zéolithe désaluminée ou sur la matrice.

Ledit métal choisi parmi les métaux des groupes IIIA, IVA et VIIB et éventuellement présent dans le catalyseur utilisé dans le procédé d'isomérisation selon l'invention est choisi parmi le gallium, l'indium, l'étain et le rhénium, de préférence parmi l'indium, l'étain et le rhénium.

La préparation du catalyseur utilisé dans le procédé d'isomérisation selon l'invention peut être effectuée par toute méthode connue de l'Homme du métier. De manière préférée, à la suite de la calcination réalisée à la fin de l'étape iii) de mise en forme, au moins un métal VIII est introduit sur le support zéolithique, à savoir soit majoritairement sur la matrice, soit majoritairement sur la zéolithe désaluminée soit encore sur l'ensemble zéolithe désaluminée-matrice. Le dépôt dudit métal sur le support zéolithique est avantageusement effectué par la technique d'imprégnation à sec, la technique d'imprégnation par excès ou par échange ionique. Lorsque plusieurs métaux sont introduits, ceux-ci peuvent être introduits soit tous de la même façon soit par des techniques différentes.

Tous les précurseurs de métaux du groupe VIII conviennent pour le dépôt d'un ou de plusieurs métal(ux) du groupe VIII sur le support zéolithique. En particulier, pour tout métal noble du groupe VIII, on peut utiliser des composés ammoniaqués ou des composés tels que par exemple le chloroplatinate d'ammonium, le dichlorure de platine dicarbonyle, l'acide hexahydroxyplatinique, le chlorure de palladium ou le nitrate de palladium. Le platine est généralement introduit sous forme d'acide hexachloroplatinique. L'introduction du métal noble du groupe VIII est de préférence effectuée par imprégnation à l'aide d'une solution aqueuse ou organique de l'un des composés métalliques cités ci-dessus. Parmi les solvants organiques utilisables, on peut citer les hydrocarbures paraffiniques, naphténiques ou aromatiques contenant par exemple de 6 à 12 atomes de carbone par molécule, et les composés organiques halogénés contenant par exemple de 1 à 12 atomes de carbone par molécule. On peut citer par exemple le n-heptane, le méthylcyclohexane, le toluène et le chloroforme. On peut aussi utiliser les mélanges de solvants.

Le contrôle de certains paramètres mis en œuvre lors du dépôt, en particulier la nature du précurseur du (des) métal(ux) du groupe VIII utilisé(s), permet d'orienter le dépôt du(es)dit(s) métal(ux) majoritairement sur la matrice ou sur la zéolithe. Ainsi, pour introduire le(s) métal(ux) du groupe VIII, préférentiellement le platine et/ou le palladium, majoritairement sur la matrice, on peut mettre en œuvre un échange anionique avec de l'acide hexachloroplatinique et/ou de l'acide hexachloropalladique, en présence d'un agent compétiteur, par exemple de l'acide chlorhydrique, le dépôt étant en général suivi d'une calcination, par exemple à une température comprise entre 350 et 550°C et pendant une durée comprise entre 1 et 4 heures. Avec de tels précurseurs, le(s) métal(ux) du groupe VIII est(sont) déposé(s) majoritairement sur la matrice et le(s)dit(s) métal(ux) présente(nt) une bonne dispersion et une bonne répartition macroscopique à travers le grain de catalyseur.

On peut aussi envisager de déposer le(s) métal(ux) du groupe VIII, préférentiellement le platine et/ou le palladium, par échange cationique de manière à ce que le(s)dit(s) métal(ux) soi(en)t déposé(s) majoritairement sur la zéolithe. Ainsi, dans le cas du platine, le précurseur peut être par exemple choisi parmi :

- les composés ammoniaqués tels que les sels de platine (II) tétrammines de formule $Pt(NH_3)_4X_2$, les sels de platine (IV) hexammines de formule $Pt(NH_3)_6X_4$ ; les sels de platine (IV) halogénopentammines de formule $(PtX(NH_3)_5)X_3$ ; les sels de platine N-tétrahalogénodiammines de formule $PtX_4(NH_3)_2$ ; et
- les composés halogénés de formule $H(Pt(acac)_2X)$ ;

X étant un halogène choisi dans le groupe formé par le chlore, le fluor, le brome et l'iode, X étant de préférence le chlore, et "acac" représentant le groupe acétylacétonate (de formule brute $C_5H_7O_2$), dérivé de l'acétylacétone. Avec de tels précurseurs, le(s) métal(ux) du groupe VIII est(sont) déposé(s) majoritairement sur la zéolithe et le(s)dit(s) métal(ux) présente(nt) une bonne dispersion et une bonne répartition macroscopique à travers le grain de catalyseur.

L'imprégnation à sec du métal du groupe VIII sur le support zéolithique conduit au dépôt dudit métal à la fois sur la matrice et sur la zéolithe désaluminée.

Dans le cas où le catalyseur utilisé dans le procédé d'isomérisation de l'invention contient également au moins un métal choisi parmi les métaux des groupes IIIA, IVA et VIIB, toutes les techniques de dépôt d'un tel métal connues de l'homme du métier et tous les précurseurs de tels métaux peuvent convenir.

On peut ajouter le(s) métal(ux) du groupe VIII et celui(ceux) des groupes IIIA, IVA et VIIB, soit séparément soit simultanément dans au moins une étape unitaire. Lorsqu'au moins un métal des groupes IIIA, IVA et VIIB est ajouté séparément, il est préférable qu'il soit ajouté après le métal du groupe VIII.

Le métal additionnel choisi parmi les métaux des groupes IIIA, IVA et VIIB peut être introduit par l'intermédiaire de composés tels que par exemple les chlorures, les bromures et les nitrates des métaux des groupes IIIA, IVA et VIIB. Par exemple dans le cas de l'indium, on utilise avantageusement le nitrate ou le chlorure et dans le cas du rhénium, on utilise avantageusement l'acide perrhénique. Dans le cas de l'étain, les chlorures d'étain $SnCl_2$ et $SnCl_4$ sont préférés.

Le métal additionnel choisi parmi les métaux des groupes IIIA, IVA et VIIB peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel choisi parmi les métaux des groupes IIIA, IVA et VIIB est introduit avant le métal du groupe VIII, le composé du métal IIIA, IVA et/ou VIIB utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.

De plus, des traitements intermédiaires tels que par exemple une calcination et/ou une réduction peuvent être appliqués entre les dépôts successifs des différents métaux.

La préparation du catalyseur se termine généralement par une calcination, habituellement à une température comprise entre 250°C et 600°C, pour une durée comprise entre 0,5 et 10 heures, de préférence précédée d'un séchage, par exemple à l'étuve, à une température allant de la température ambiante à 250°C, de préférence de 40°C à 200°C. Ladite étape de séchage est de préférence menée pendant la montée en température nécessaire pour effectuer ladite calcination. On peut mettre en oeuvre une réduction préalable du catalyseur *ex situ,* sous courant d'hydrogène, par exemple à une température de 450°C à 600°C, pendant une durée de 0,5 à 4 heures.

Le dépôt du(es)dit(s) métal(ux) du groupe VIII est avantageusement opéré de façon telle que la dispersion du(des)dit(s) métal(ux), déterminée par chimisorption, soit de 50 % à 100 %, de préférence de 60 % à 100 % et, de manière encore plus préférée, de 70 % à 100 %. Le dépôt du(es)dit(s) métal(ux) du groupe VIII est également avantageusement opéré de manière à obtenir une bonne répartition du(des)dit(s) métal(ux) dans le catalyseur mis en forme. Cette répartition est caractérisée par son profil obtenu par microsonde de Castaing. Le rapport des concentrations de chaque élément du groupe VIII au cœur du grain par rapport au bord de ce même grain, défini comme étant le coefficient de répartition, est avantageusement de 0,7:1 à 1,3:1, de préférence de 0,8:1 à 1,2:1.

Dans la composition du catalyseur utilisé dans le procédé d'isomérisation selon l'invention, la zéolithe de type structural EUO désaluminée, de préférence la zéolithe EU-1 désaluminée, entre plus particulièrement à raison de 1 à 90 %, de préférence de 3 à 80 % et, de manière encore plus préférée, de 4 à 60 % en poids par rapport au poids de catalyseur. Le(s) métal(ux) du groupe VIII, de préférence le platine, déposé(s) sur la zéolithe et/ou sur la matrice, représente(nt) de 0,01 à 4 %, de préférence de 0,05 à 2,0 %, en poids par rapport au poids de catalyseur. La matrice constitue le complément à 100 %. Lorsque ledit catalyseur contient au moins un métal choisi parmi les métaux des groupes IIIA, IVA et VIIB, la teneur en celui-ci peut aller jusqu'à 2 % en poids par rapport au poids de catalyseur. Elle est alors avantageusement de 0,01 à 2 %, de préférence de 0,05 à 1,0 % en poids.

Lorsque ledit catalyseur contient du soufre, la teneur en celui-ci peut être telle que le rapport du nombre d'atomes de soufre sur le nombre d'atomes de métal du groupe VIII déposés aille jusqu'à 2:1. Il est alors avantageusement de 0,5:1 à 2:1. La zéolithe de type structural EUO désaluminée, de préférence la zéolithe EU-1 désaluminée, présente dans le catalyseur employé pour la mise en oeuvre du procédé d'isomérisation selon l'invention se présente très préférentiellement sous sa forme protonée (forme hydrogène $H^+$) dans laquelle la proportion en cation autre que $H^+$ est inférieure à 30% du nombre total de cations, de préférence inférieure à 20 % et de manière très préférée inférieure à 10 % et de manière encore plus préférée inférieure à 5% par rapport au nombre total de cations sur la zéolithe. Cette forme protonée est généralement obtenue lors du traitement thermique en présence de vapeur d'eau réalisé pour la mise en oeuvre desdits deuxième et troisième modes de réalisation de ladite étape ii), ou lors de l'étape de calcination subséquente à la mise en forme de la zéolithe désaluminée avec une matrice.

Dans le cas où le catalyseur ne contient pas de soufre, une réduction du métal sous hydrogène est avantageusement réalisée *in situ* avant injection de la charge.

Dans le cas où le catalyseur utilisé dans l'invention contient du soufre, le soufre est introduit sur le catalyseur mis en forme, calciné, contenant le ou les métaux cités précédemment, soit *in situ* avant la réaction catalytique, soit *ex situ.* La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

Le procédé d'isomérisation selon l'invention consiste à mettre en contact une coupe aromatique contenant au moins un composé aromatique ayant huit atomes de carbone par molécule avec au moins ledit catalyseur contenant au moins ladite zéolithe de type structural EUO désaluminée, de préférence ladite zéolithe EU-1 désaluminée, ledit catalyseur ayant été préparé conformément à la mise en oeuvre de chacune desdites étapes i), ii), iii) et iv) décrites plus haut dans la présente description.

Ladite coupe aromatique contenant au moins un composé aromatique ayant huit atomes de carbone par molécule comprend en particulier comme composé aromatique ayant huit atomes de carbone par molécule soit uniquement un mélange de xylènes, soit uniquement de l'éthylbenzène, soit un mélange de xylène(s) et d'éthylbenzène. Ledit procédé d'isomérisation est mis en oeuvre généralement selon les conditions opératoires suivantes :

- une température de 300°C à 500°C, de préférence de 320°C à 450°C et de manière encore plus préférée de 340°C à 430°C ;
- une pression partielle d'hydrogène de 0,3 à 1,5 MPa, de préférence de 0,4 et 1,2 MPa et de manière encore préférée de 0,7 à 1,2 MPa ;
- une pression totale de 0,45 à 1,9 MPa, de préférence de 0,6 à 1,5 MPa ; et
- une vitesse spatiale d'alimentation, exprimée en kilogramme de charge introduite par kilogramme de catalyseur et par heure, de 0,25 à 30 $h^{-1}$, de préférence de 1 à 10 $h^{-1}$ et de manière encore préférée de 2 à 6 $h^{-1}$.

Les exemples qui suivent illustrent l'invention sans pour autant en limiter la portée.

**Exemple 1** : Préparation d'une zéolithe EU-1 désaluminée

La matière première utilisée est une zéolithe EU-1, brute de synthèse, comprenant le structurant organique à savoir le 1,6 N,N,N,N',N',N'-hexaméthylhexaméthylène diammonium et qui possède un rapport atomique Si/Al global égal à 15,3 et une teneur pondérale en sodium correspondant à un rapport atomique Na/Al (en %) égal à 30,8. Cette zéolithe a été synthétisée conformément à l'enseignement du brevet EP-B1-0.042.226. Pour la préparation d'une telle zéolithe, le mélange réactionnel présente la composition molaire suivante : 60 $SiO_2$ : 10,6 $Na_2O$ : 5,27 NaBr : 1,5 $Al_2O_3$ : 19,5 Hexa-$Br_2$ : 2777 $H_2O$. Hexa-$Br_2$ étant le 1,6 N,N,N,N',N',N'-hexaméthylhexaméthylène diammonium, le brome étant le contre-ion. Le mélange réactionnel est placé dans un autoclave sous agitation (300 tours/min) pendant 5 jours à 180°C.

Cette zéolithe EU-1 subit tout d'abord une calcination dite sèche à 550°C sous flux d'air sec durant 24 heures de manière à éliminer le structurant organique. Puis le solide obtenu est soumis à quatre échanges ioniques dans une solution de $NH_4NO_3$ 10N, à environ 100°C pendant 4 heures pour chaque échange. Le solide ainsi obtenu est référencé EU-1(1) et possède un rapport atomique Si/Al global = 15,3 et un rapport atomique Na/Al=0,51%.

La zéolithe EU-1 est alors soumise à 3 attaques acides successives, à l'aide d'acide nitrique 2N, 5N puis 8N, à environ 100°C, pendant 4 heures. Le volume V de la solution d'acide nitrique engagé (en ml) est égal à 10 fois le poids P (en gramme) de zéolithe EU-1 sèche (V/P=10). Entre chacune des attaques acides, la zéolithe est lavée à l'eau. Ensuite, la zéolithe est séchée une nuit à 120°C.

A l'issue de ces traitements, on obtient la zéolithe EU-1(2) qui possède un rapport atomique Si/Al global, mesuré par fluorescence X, égal à 21,35 et un rapport atomique Na/Al inférieur à 0,2%. Ainsi, 28,3 % poids des atomes d'aluminium présents dans la zéolithe EU-1 brute de synthèse ont été extraits.

**Exemple 2 (non conforme)** : Préparation du catalyseur A comprenant une zéolithe EU-1 non désaluminée

La zéolithe EU-1(1) obtenue à l'exemple 1 est ensuite mise en forme par extrusion avec un gel d'alumine de manière à obtenir, après séchage à une température égale à 100 °C pendant 1 nuit et une calcination sous air sec menée à une température égale à 450°C pendant 4 heures, le support S1 qui contient en poids 15 % de zéolithe EU-1 et 85 % d'alumine.

Ce support S1 est soumis à un échange anionique avec de l'acide hexachloroplatinique en présence d'acide chlorhydrique en tant qu'agent compétiteur, de manière à déposer 1 % poids de platine par rapport au poids du catalyseur. Le solide humide est ensuite séché à 120°C pendant 12 heures et calciné sous un débit d'air sec à la température de 500°C pendant une heure.

Le catalyseur A ainsi obtenu contient, en poids, 15 % de zéolithe EU-1, 84 % d'alumine et 1 % de platine.

**Exemple 3 (invention)** : Préparation du catalyseur B comprenant une zéolithe EU-1 désaluminée

La zéolithe EU-1(2) désaluminée obtenue à l'exemple 1 est ensuite mise en forme par extrusion avec un gel d'alumine de manière à obtenir, après séchage à une température égale à 100°C pendant une nuit et une calcination sous air sec menée à une température égale à 450°C pendant 4 heures, le support S2 qui contient en poids 15 % de zéolithe EU-1 désaluminée et 85 % d'alumine.

Ce support S2 est soumis à un échange anionique avec de l'acide hexachloroplatinique en présence d'acide chlorhydrique en tant qu'agent compétiteur, de manière à déposer 1 % poids de platine par rapport au catalyseur. Le solide humide est ensuite séché à 120°C pendant 12 heures et calciné sous un débit d'air sec à la température de 500°C pendant une heure.

Le catalyseur B ainsi obtenu contient, en poids, 15 % de zéolithe EU-1 désaluminée, 84 % d'alumine et 1 % de platine.

**Exemple 4 (non conforme)** : Préparation du catalyseur C comprenant une zéolithe EU-1 non désaluminée

La matière première utilisée est une zéolithe EU-1 brute de synthèse, comprenant le structurant organique à savoir le 1,6 N,N,N,N',N',N'-hexaméthylhexaméthylène diammonium et qui possède un rapport atomique Si/Al global égal à 21,35, une teneur pondérale en sodium correspondant à un rapport atomique Na/Al (en %) égal à 27,4. Cette zéolithe a été synthétisée conformément à l'enseignement du brevet EP-B1-0.042.226. Pour la préparation d'une telle zéolithe, le mélange réactionnel présente la composition molaire suivante : 60 $SiO_2$ : 10,6 $Na_2O$ : 5,27 NaBr : 1 $Al_2O_3$ : 19,5 Hexa-$Br_2$ : 2777 $H_2O$. Hexa-$Br_2$ étant le 1,6 N,N,N,N',N',N'-hexaméthylhexaméthylène diammonium, le brome étant le contre-ion. Le mélange réactionnel est placé dans un autoclave sous agitation (300 tours/min) pendant 4 jours à 180°C.

Cette zéolithe EU-1 subit tout d'abord une calcination dite sèche à 550°C sous flux d'air sec durant 24 heures. Puis le solide obtenu est soumis à quatre échanges ioniques dans une solution de $NH_4NO_3$ 10N, à environ 100°C pendant 4 heures pour chaque échange. Le solide ainsi obtenu est référencé EU-1(3) et possède un rapport atomique Si/Al global = 21,35 et un rapport atomique Na/Al = 0,52%.

La zéolithe EU-1(3) est ensuite mise en forme par extrusion avec un gel d'alumine de manière à obtenir, après séchage à 100°C pendant une nuit et calcination sous air sec à 450°C pendant 4 heures, le support S3 qui contient en poids 15 % de zéolithe EU-1 et 85 % d'alumine.

Ce support S3 est soumis à un échange anionique avec de l'acide hexachloroplatinique en présence d'acide chlorhydrique en tant qu'agent compétiteur, de manière à déposer 1 % poids de platine par rapport au catalyseur. Le solide humide est ensuite séché à 120°C pendant 12 heures et calciné sous un débit d'air sec à la température de 500°C pendant une heure.

Le catalyseur C ainsi obtenu contient, en poids, 15 % de zéolithe EU-1, 84 % d'alumine et 1 % de platine.

**Exemple 5** : Évaluation des propriétés catalytiques des catalyseurs A, B et C en isomérisation de l'ethylbenzène.

La charge à isomériser, mise en contact avec le catalyseur A, le catalyseur B puis le catalyseur C, est constituée uniquement d'éthylbenzène.

Les conditions opératoires de l'isomérisation sont les suivantes :

- température : 410°C ;
- pression totale : 10 bar (1 bar = 0,1 MPa) ;
- pression partielle d'hydrogène : 8 bar.
- charge : ethylbenzène
- vitesse spatiale d'alimentation, exprimée en kilogramme de charge introduite par kilogramme de catalyseur et par heure, égale à 8,7 $h^{-1}$.

On évalue successivement les propriétés catalytiques des catalyseurs A, B et C pour l'isomérisation de l'éthylbenzène. Chacun des catalyseurs A, B et C est réduit sous hydrogène pendant 4 heures à 480°C avant injection de la charge.

Les catalyseurs ont été évalués en terme de sélectivité. En plus de la réaction désirée correspondant à l'isomérisation de l'éthylbenzène qui conduit à des xylènes, des réactions parasites conduisent à trois types de sous-produits : les paraffines résultant essentiellement de réactions d'ouverture de cycles naphténiques suivies de craquage, les aromatiques formés par réactions de dismutation et de transalkylation des aromatiques à 8 atomes de carbone (AC8), et enfin les naphtènes dont les naphtènes à 8 atomes de carbone (N8) formés par l'hydrogénation des aromatiques. Les N8 pouvant être recyclés, on compare les sélectivités des produits autres que les naphtènes.

La sélectivité envers les différents produits de la réaction d'isomérisation est calculée au moyen des rendements de ces différents produits. Les rendements sont déterminées à partir des % massiques des différents produits obtenus par analyse des effluents et sont calculés comme suit :

Rendement en isomérisation : $\sum$ xylènes

$$\text{Rendement en dismutation :} \quad 2 \times \frac{106}{134} \times DEB \text{ (diéthylbenzène)}$$

$$\text{Rendement en désalkylation :} \quad \frac{106}{30} \times C_2 \text{ (éthane)}$$

Rendement en transalkylation : $\frac{106}{134} \times DMEB$ (diméthyléthylbenzène) + $\frac{106}{120} \times ET$ (éthyltoluène)

Rendement en naphtènes : $\frac{106}{84} \times \sum N_6$ (naphtènes à 6 carbones) + $\frac{106}{98} \times \sum N_7$ (naphtènes à 7 carbones) + $\frac{106}{112} \times \sum N_8$ (naphtènes à 8 carbones)

Rendement en produits de craquage : $\sum C_3 + C_4 + C_5 + C_6$ ($C_3$-$C_6$ aliphatiques)

$$\text{La sélectivité en produit i autre que naphtènes} = \frac{rendement\ en\ produit\ i}{\sum rendement - rendement\ en\ napthènes}$$

Tableau 1 : Sélectivité des produits de réaction (%) autres que les naphtènes sur les catalyseurs A, B et C après 4000 min de réaction

| Sélectivité (%) | Catalyseur A | Catalyseur B | Catalyseur C |
|---|---|---|---|
| Isomérisation | 68,1 | 73,0 | 64,3 |
| Dismutation | 16,3 | 12,2 | 18,4 |
| Désalkylation | 5,0 | 5,0 | 5,5 |
| Craquage | 9,6 | 7,8 | 10,6 |
| Transalkylation | 1.0 | 1,0 | 1,2 |

Les résultats présentés dans le tableau 1 montrent que le catalyseur B comprenant une zéolithe EU-1 désaluminée dont au moins 10% massique des atomes d'aluminium ont été extraits de la zéolithe EU-1 brute de synthèse conduit à de bien meilleures performances catalytiques en terme de sélectivité que celles obtenues au moyen des catalyseurs A et C comprenant une zéolithe EU-1 non désaluminée. En particulier, l'utilisation du catalyseur B pour l'isomérisation de l'éthylbenzène conduit à une amélioration sensible de la sélectivité en isomérisation aux dépens de la sélectivité en dismutation, désalkylation, transalkylation et craquage.

**Revendications**

1. Procédé d'isomérisation d'une coupe aromatique contenant au moins un composé aromatique ayant huit atomes de carbone par molécule à une température comprise entre 300 et 500°C, une pression partielle d'hydrogène comprise entre 0,3 et 1,5 MPa, une pression totale comprise entre 0,45 et 1,9 MPa et une vitesse spatiale d'alimentation, exprimée en kilogramme de charge introduite par kilogramme de catalyseur et par heure, comprise entre 0,25 et 30 h$^{-1}$, dans lequel on met en contact un catalyseur contenant au moins une zéolithe de type structural EUO et ladite coupe aromatique ledit catalyseur étant préparé selon un procédé comprenant au moins les étapes suivantes :

   i) la synthèse d'au moins une zéolithe de type structural EUO ayant un rapport atomique Si/Al global compris entre 5 et 45,
   ii) la désalumination de la zéolithe obtenue à l'issue de ladite étape i), par au moins un traitement par une solution aqueuse d'un acide minéral ou d'un acide organique, de telle manière qu'au moins 10% massique des atomes d'aluminium soient extraits de ladite zéolithe issue de ladite étape i),
   iii) la mise en forme de ladite zéolithe désaluminée avec une matrice,
   iv) le dépôt d'au moins un métal du groupe VIII de la classification périodique des éléments, l'ordre de réalisation desdites étapes iii) et iv) étant indifférent à la suite de ladite étape ii).

2. Procédé d'isomérisation selon la revendication 1 tel que ladite zéolithe de type structural EUO présente dans ledit catalyseur est une zéolithe EU-1.

3. Procédé d'isomérisation selon la revendication 1 ou la revendication 2 tel que ladite zéolithe de type structural EUO obtenue à l'issue de ladite étape i) présente un rapport atomique Si/Al global compris entre 10 et 25.

4. Procédé d'isomérisation selon l'une des revendications 1 à 3 tel que ladite étape ii) consiste à extraire au moins 20% massique des atomes d'aluminium de ladite zéolithe de type structural EUO issue de ladite étape i).

5. Procédé d'isomérisation selon l'une des revendications 1 à 4 tel que pour la mise en œuvre de ladite étape ii), la zéolithe de type structural EUO issue de ladite étape i) est soumise à une calcination sous flux d'air sec, à une température comprise entre 400 et 600°C puis est soumise à au moins un traitement par une solution aqueuse d'un acide minéral ou d'un acide organique.

6. Procédé d'isomérisation selon la revendication 5 tel que un ou plusieurs échange(s) ionique(s) par au moins une solution $NH_4NO_3$ est(sont) opéré(s) entre la calcination sous flux d'air sec et le traitement par ladite solution aqueuse acide.

7. Procédé d'isomérisation selon la revendication 5 ou 6 tel que le traitement de la zéolithe par une solution aqueuse d'un acide minéral ou d'un acide organique est réalisé à une température comprise entre 30°C et 120°C, la concentration de l'acide dans ladite solution aqueuse étant comprise entre 0,05 et 20 mol.l$^{-1}$, le rapport entre le volume de solution acide en ml et le poids de zéolithe traitée en g étant compris entre 1 et 50, la durée de l'attaque acide étant supérieure à 1 heure.

8. Procédé d'isomérisation selon l'une des revendications 5 à 7 tel que ledit acide est un acide minéral choisi parmi l'acide nitrique, l'acide chlorhydrique et l'acide sulfurique.

9. Procédé d'isomérisation selon l'une des revendications 5 à 8 tel que le nombre de traitement successif de la zéolithe par une solution aqueuse acide est inférieur à 4.

10. Procédé d'isomérisation selon l'une des revendications 1 à 4 tel que pour la mise en oeuvre de ladite étape ii), la zéolithe de type structural EUO, issue de ladite étape i), est soumise à une calcination sous flux d'air sec, à une température comprise entre 400 et 600°C puis à un ou plusieurs échange(s) ionique(s) par au moins une solution $NH_4NO_3$ puis est soumise à au moins un cycle de désalumination de la charpente zéolithique comportant au moins un traitement thermique réalisé en présence de vapeur d'eau et au moins une attaque acide par au moins une solution aqueuse d'un acide minéral ou organique.

11. Procédé d'isomérisation selon l'une des revendications 1 à 4 tel que pour la mise en oeuvre de ladite étape ii), la zéolithe de type structural EUO, issue de ladite étape i), est soumise à un traitement thermique réalisé en présence de vapeur d'eau à une température comprise entre 450 et 850°C, puis à au moins un traitement par une solution aqueuse d'un acide minéral ou organique.

12. Procédé d'isomérisation selon l'une des revendications 1 à 11 tel que ladite étape iii) précède ladite étape iv).

13. Procédé d'isomérisation selon l'une des revendications 1 à 12 tel que ladite matrice employée dans l'étape iii) est une alumine.

14. Procédé d'isomérisation selon l'une des revendications 1 à 13 tel que ledit métal du groupe VIII employé pour la mise en oeuvre de ladite étape iv) est le platine.

15. Procédé d'isomérisation selon l'une des revendications 1 à 14 tel que au moins un métal choisi parmi les métaux des groupes IIIA, IVA et VIIB est introduit pour la mise en oeuvre de ladite étape iv).

**Patentansprüche**

1. Verfahren zur Isomerisierung einer aromatischen Fraktion, die mindestens eine aromatische Verbindung mit acht Kohlenstoffatomen pro Molekül aufweist, bei einer Temperatur im Bereich von 300 bis 500 °C, einem Wasserstoffpartialdruck im Bereich von 0,3 bis 1,5 MPa, einem Gesamtdruck im Bereich von 0,45 bis 1,9 MPa und einer raumbezogenen Zufuhrgeschwindigkeit, ausgedrückt in Kilogramm an Beschickungsgut, das pro Kilogramm an Katalysator und pro Stunde eingeleitet wird, im Bereich von 0,25 bis 30 h$^{-1}$, wobei ein Katalysator, der mindestens einen Zeolith des Strukturtyps EUO enthält, mit der aromatischen Fraktion in Kontakt gebracht wird, wobei der Katalysator gemäß einem Verfahren hergestellt wird, das mindestens die folgenden Schritte umfasst:

i) Synthese mindestens eines Zeoliths vom Strukturtyp EUO mit einem Gesamtwert des Si/Al-Atomverhältnisses im Bereich von 5 bis 45,
ii) Aluminiumabreicherung des Zeoliths, der nach Abschluss des Schrittes i) erhalten wurde, durch mindestens eine Behandlung mit einer wässrigen Lösung einer Mineralsäure oder einer organischen Säure, derart, dass mindestens 10 Massen-% der Aluminiumatome aus dem Zeolith extrahiert werden, welcher aus dem Schritt i) stammt,
iii) Formgebung des aluminiumabgereicherten Zeoliths mit einer Matrix,
iv) Abscheiden mindestens eines Metalls der Gruppe VIII des Periodensystems der Elemente, wobei nach dem Schritt ii) die Schritte iii) und iv) in beliebiger Reihenfolge durchgeführt werden können.

2. Verfahren zur Isomerisierung nach Anspruch 1, derart, dass es sich bei dem Zeolith des Strukturtyps EUO, welcher in dem Katalysator vorliegt, um einen EU-1-Zeolith handelt.

3. Verfahren zur Isomerisierung nach Anspruch 1 oder Anspruch 2, derart, dass der Zeolith des Strukturtyps EUO, welcher nach Abschluss des Schrittes i) erhalten wird, einen Gesamtwert des Si/Al-Atomverhältnisses im Bereich von 10 bis 25 aufweist.

4. Verfahren zur Isomerisierung nach einem der Ansprüche 1 bis 3, derart, dass der Schritt ii) darin besteht, mindestens 20 Massen-% der Aluminiumatome des Zeoliths vom Strukturtyp EUO, welcher aus dem Schritt i) stammt, zu extrahieren.

5. Verfahren zur Isomerisierung nach einem der Ansprüche 1 bis 4, derart, dass zur Durchführung des Schrittes ii) der Zeolith des Strukturtyps EUO, welcher aus dem Schritt i) stammt, einem Brennvorgang unter einem trockenen Luftstrom unterzogen wird, bei einer Temperatur im Bereich von 400 bis 600 °C, woraufhin er mindestens einer Behandlung mit einer wässrige Lösung einer Mineralsäure oder einer organischen Säure unterzogen wird.

6. Verfahren zur Isomerisierung nach Anspruch 5, derart, dass zwischen dem Brennvorgang unter einem trockenen Luftstrom und der Behandlung mit der sauren wässrigen Lösung ein oder mehrere Ionenaustausch-Vorgang/Vorgänge mittels mindestens einer $NH_4NO_3$-Lösung vorgenommen wird/werden.

7. Verfahren zur Isomerisierung nach Anspruch 5 oder 6, derart, dass die Behandlung des Zeoliths mit einer wässrigen Lösung einer Mineralsäure oder einer organischen Säure bei einer Temperatur im Bereich von 30 °C bis 120 °C durchgeführt wird, wobei die Konzentration der Säure in der wässrigen Lösung im Bereich von 0,05 bis 20 mol.l$^{-1}$

liegt, das Verhältnis zwischen dem Volumen der sauren Lösung in ml unde dem Gewicht des behandelten Zeoliths in g im Bereich von 1 bis 50 liegt, die Dauer der Säureeinwirkung mehr als 1 Stunde beträgt.

8. Verfahren zur Isomerisierung nach einem der Ansprüche 5 bis 7, derart, dass es sich bei der Säure um eine Mineralsäure handelt, die aus Salpetersäure, Salzsäure und Schwefelsäure ausgewählt ist.

9. Verfahren zur Isomerisierung nach einem der Ansprüche 5 bis 8, derart, dass die Anzahl an aufeinanderfolgenden Behandlungen des Zeoliths mit einer sauren wässrigen Lösung weniger als 4 beträgt.

10. Verfahren zur Isomerisierung nach einem der Ansprüche 1 bis 4, derart, dass zur Durchführung des Schrittes ii) der Zeolith des Strukturtyps EUO, welcher aus dem Schritt i) stammt, einem Brennvorgang unter einem trockenen Luftstrom unterzogen wird, bei einer Temperatur im Bereich von 400 bis 600 °C, woraufhin er einem oder mehreren Ionenaustausch-Vorgang/Vorgängen mittels mindestens einer $NH_4NO_3$-Lösung unterzogen wird, um anschließend mindestens einem Zyklus der Aluminiumabreicherung des Zeolithgerüsts unterzogen zu werden, der mindestens eine Wärmebehandlung, welche in Gegenwart von Wasserdampf durchgeführt wird, und mindestens eine Säureeinwirkung durch mindestens eine wässrigen Lösung einer mineralischen oder organischen Säure aufweist.

11. Verfahren zur Isomerisierung nach einem der Ansprüche 1 bis 4, derart, dass zur Durchführung des Schrittes ii) der Zeolith des Strukturtyps EUO, welcher aus dem Schritt i) stammt, einer Wärmebehandlung unterzogen wird, welche in Gegenwart von Wasserdampf bei einer Temperatur im Bereich von 450 bis 850 °C durchgeführt wird, woraufhin er mindestens einer Behandlung mit einer wässrigen Lösung einer Mineralsäure oder einer organischen Säure unterzogen wird.

12. Verfahren zur Isomerisierung nach einem der Ansprüche 1 bis 11, derart, dass der Schritt iii) dem Schritt iv) vorangeht.

13. Verfahren zur Isomerisierung nach einem der Ansprüche 1 bis 12, derart, dass es sich bei der Matrix, die im Schritt iii) zum Einsatz kommt, um ein Aluminiumoxid handelt.

14. Verfahren zur Isomerisierung nach einem der Ansprüche 1 bis 13, derart, dass es sich bei dem Metall der Gruppe VIII, welches zur Durchführung des Schrittes iv) verwendet wird, um Platin handelt.

15. Verfahren zur Isomerisierung nach einem der Ansprüche 1 bis 14, derart, dass mindestens ein Metall, welches aus den Metallen der Gruppen IIIA, IVA und VIIB ausgewählt ist, zur Durchführung des Schrittes iv) beigefügt wird.

## Claims

1. A process for isomerizing an aromatic cut containing at least one aromatic compound containing eight carbon atoms per molecule, at a temperature in the range 300 to 500°C, a hydrogen partial pressure in the range 0.3 to 1.5 MPa, at total pressure in the range 0.45 to 1.9 MPa and a feed space velocity, expressed in kilograms of feed introduced per kilogram of catalyst per hour, in the range 0.25 to 30h$^{-1}$, in which a catalyst containing at least one zeolite with structure type EUO is brought into contact with said aromatic cut, said catalyst having been prepared using a process comprising at least the following steps:

   i) synthesizing at least one zeolite with structure type EUO having an overall Si/Al atomic ratio in the range 5 to 45;
   ii) dealuminating the zeolite obtained at the end of said step i) using at least one treatment with an aqueous solution of a mineral acid or an organic acid, such that at least 10% by weight of the aluminium atoms are extracted from said zeolite resulting from said step i);
   iii) forming said dealuminated zeolite with a matrix;
   iv) depositing at least one metal from group VIII of the periodic table of the elements, the order of carrying out said steps iii) and iv) being inconsequential following on from said step ii).

2. The isomerization process according to claim 1, in which said zeolite with structure type EUO present in said catalyst is an EU-1 zeolite.

3. The isomerization process according to claim 1 or claim 2, in which said zeolite with structure type EUO obtained at the end of said step i) has an overall Si/Al atomic ratio in the range 10 to 25.

**4.** The isomerization process according to one of claims 1 to 3, in which said step ii) consists of extracting at least 20% by weight of aluminium atoms from said zeolite with structure type EUO resulting from said step i).

**5.** The isomerization process according to one of claims 1 to 4, in which in order to carry out said step ii), the zeolite with structure type EUO resulting from said step i) undergoes calcining in a stream of dry air at a temperature in the range 400°C to 600°C then undergoes at least one treatment with an aqueous solution of a mineral acid or an organic acid.

**6.** The isomerization process according to claim 5, in which one or more ion exchange step(s) using at least one $NH_4NO_3$ solution is (are) carried out between the calcining in a stream of dry air and the treatment using said aqueous acid solution.

**7.** The isomerization process according to claim 5 or claim 6, in which treatment of the zeolite with an aqueous solution of a mineral acid or an organic acid is carried out at a temperature in the range 30°C to 120°C, the concentration of the acid in said aqueous solution being in the range 0.05 to 20 mol/$l^{-1}$, the ratio between the volume of the acid solution in ml and the weight of the zeolite treated in g being in the range 1 to 50, the duration of the acid attack being more than 1 hour.

**8.** The isomerization process according to one of claims 5 to 7, in which said acid is a mineral acid selected from nitric acid, hydrochloric acid and sulphuric acid.

**9.** The isomerization process according to one of claims 5 to 8, in which the number of successive treatments of the zeolite with an aqueous acidic solution is less than 4.

**10.** The isomerization process according to one of claims 1 to 4, in which in order to carry out said step ii), the zeolite with structure type EUO resulting from said step i) undergoes calcining in a stream of dry air at a temperature in the range 400°C to 600°C then undergoes one or more ion exchange (s) using at least one $NH_4NO_3$ solution, then undergoes at least one cycle for dealumination of the zeolitic framework comprising at least one heat treatment carried out in the presence of steam and at least one acid attack using at least one aqueous solution of a mineral or organic acid.

**11.** The isomerization process according to one of claims 1 to 4 in which, in order to carry out said step ii), the zeolite with structure type EUO resulting from said step i) undergoes heat treatment carried out in the presence of steam at a temperature in the range 450°C to 850°C then undergoes at least one treatment with an aqueous solution of a mineral or organic acid.

**12.** The isomerization process according to one of claims 1 to 11, in which said step iii) precedes said step iv).

**13.** The isomerization process according to one of claims 1 to 12, in which said matrix used in step iii) is an alumina.

**14.** The isomerization process according to one of claims 1 to 13, in which said group VIII metal used to carry out said step iv) is platinum.

**15.** The isomerization process according to one of claims 1 to 14, in which at least one metal selected from metals from groups IIIA, IVA and VIIB is introduced to carry out said step iv).

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 4467129 A
- US 4482773 A
- EP 13617 B
- US 4723051 A
- US 4665258 A
- FR 2477903 A
- EP 923987 A1
- WO 2005065380 A
- EP 0042226 A
- EP 0051318 A
- EP 0159845 A
- US 4640829 A
- EP 0042226 B1

### Littérature non-brevet citée dans la description

- **CH. BAERLOCHER ; W.M. MEIER ; D.H. OISON.** Atlas of Zeolite Framework Types. 2001
- **N.A. BRISCOE et al.** *ont enseigné dans un article de la revue Zeolites,* 1988, vol. 8, 74